# EUROPEAN PATENT SPECIFICATION

(11) **EP 3 018 601 B1**
(45) Date of publication and mention of the grant of the patent: **23.06.2021**
(21) Application number: 14192498.5
(22) Date of filing: 10.11.2014
(51) Int. Cl.: A61M 1/34, A61M 1/36

(54) **Blood purification device graphical user interface method**
Verfahren für eine graphische Benutzerschnittstelle einer Blutreinigungsvorrichtung
Procédé de dispositif de purification du sang

(43) Date of publication of application: 11.05.2016
(73) Proprietor: B. Braun Avitum AG, 34212 Melsungen (DE)
(72) Inventor: Bocz, Máté, 1094 Budapest (HU); Béky, Zsófia, 1037 Budapest (HU)
(74) Representative: Winter, Brandl - Partnerschaft mbB

(56) References cited:
- WO-A1-2014/033119
- WO-A2-2008/074316
- US-A- 5 487 827
- US-A1- 2012 138 533
- US-A1- 2014 325 447
- US-B1- 7 719 542

## Description

The invention relates generally to blood purification and renal replacement therapy. More specifically, the invention relates to monitoring device operation during blood purification and renal replacement therapy, and to generate user feedback based on an analysis of the monitored device operation.

Blood purification devices are complex systems and as such combinations of hardware and software components. Operators of such machines are often not aware of what they are required and/or expected to do next, whether or not the machine needs interaction, and if so, how to interact.

Among known methods and systems, a device may be designed to include a number of functions for controlling parameter values, i.e. parameter control functions, to enable a user to quickly and easily control variable parameter values using a graphical user interface and to enable the user to obtain the desired precision of control of such variables. One such system provides e.g. four control functions: a data entry function, single step function, a scroll function, and a translation function. The user can select the particular control function used to control a parameter value in light of the particular amount or type of control that needs to be accomplished.

Another known medical apparatus comprises a user interface for setting parameters and includes a screen for visualizing values of said parameters, a main control unit connected to the interface, a first memory and a video memory both connected to the main control unit for storing data corresponding to images on screen. The main control unit allows setting of a new value for a parameter, displays the new value on a screen region, stores the new value in the first memory, captures from the video memory data representative of said screen region, and verifies from said representative data if the displayed value corresponds to the value in the first memory.

A further known arrangement includes an enclosure, a medical therapy machine component located inside the enclosure, a video monitor supported by the enclosure and displaying at least one of information and indicia relating to the medical therapy, an ambient light sensor positioned with respect to the enclosure so as to be able to sense a level of ambient light impinging the enclosure, and a logic implementer configured to control a level of backlight brightness for the video monitor based on the level of ambient light sensed by the sensor.

While, accordingly, known medical devices often employ icons, sound, animations, and the like on a graphical user interface to give feedback to the operator on the status of the device elements, the graphical user interfaces of device for renal replacement therapies can be quite complex and crowded since the devices themselves are complicated. The graphical user interface displays important information and provides means for the operator to trigger events. Having too many elements on screen at once can, however, result in low usability of the device. It is therefore important that the operator can instantly find all the information he is looking for, and is supported by the device on what he needs to do.

WO 2008/074316 A2 discloses a method that entails the user of a blood treatment device being guided through the individual steps without being faced by too many selection options. The user is still able to react in a flexible way to certain treatment situations. On a screen, several steps to be carried out next are shown in display fields. At least one of these display fields is provided with an identifier and is highlighted in relation to the other display fields. A control means thus provides a recommendation for the selection of the step that is to be carried out next, without forcing the user to carry out this step. The user also has the possibility of selecting one of the other steps. This permits flexibility in the selection of the steps to be carried out, which steps can be adapted to the needs of the particular patient and to the requirements of the user.

WO 2014/033119 A1 discloses an apparatus for performing a plurality of operation steps of a dialysis process. The apparatus comprises a process controller for controlling the apparatus to perform the operation steps of the dialysis process, monitor process progress of the dialysis process and monitor sensor inputs of sensors of the apparatus; and a user interface, comprising a display, an input device and a user interface, Ul, controller, wherein the UI controller is connected to enable presentation of graphical data on the display, and wherein the UI controller is connected to enable user interaction with the graphical data and connected to exchange information with the process controller, wherein the information is based on the user interaction of the user interface and monitoring of process progress of the dialysis process and sensor inputs of sensors of the apparatus by the process controller. Each of the operation steps are classified as one of a sequential operation step, which is dependent on completion of another operation step, and a non-sequential operation step, which is independent of completion of another operation step

US 5 487 827 A discloses methods and apparatuses for kidney dialysis. These include checking of dialysate bypass status using flow measurement; using a flow sensor to confirm the absence of ultrafiltration during bypass; automatic testing of ultrafiltration function by removal of a discrete volume from a portion of the dialysate flow path coupled with a pressure test of that part of the flow path; using a touch screen user interface; bar graph profile programming of ultrafiltration, sodium, and bicarbonate parameters; using a RAM card to upload treatment instructions to, and to download treatment data from, the machine; automatic setting of proportioning mode (acetate or bicarbonate) based on connections of concentrate lines; predicting dialysate conductivity values based on brand and formulation of concentrates; minimizing no-flow dead time between dialysate pulses; initiating operation in a timed mode from a machine power-off condition; preserving machine mode during machine power-fail condition; calibration scheduling and reminding; automatic level adjusting; and blood leak flow rate detecting.

US 2012/138 533 A1 discloses systems and methods of using such systems including a dialysis system and involve presenting information on a user interface for interaction by a user.

Arrangements of the exemplified known kind do not motivate any suitable concept in a blood purification device toward providing enough feedback on whether a user interaction is required, and which user interface element is concerned, with a view to improved user confidence and usability.

In view of the above, an object of the invention resides in providing a renal replacement therapy and/or blood purification device and a method therefor that are capable of providing sufficient guidance to an operator regarding user action the device is expecting from him.

In addition, the invention shall provide a method for drawing a user's attention to specific elements of the user interface and helping him even on a crowded screen.

According to the invention, this object is accomplished by a method for providing feedback in a blood purification device as defined in claim 1 and by a blood purification device arranged to carry out the method as defined in claim 9. Advantageous further developments of the invention are subject of the accompanying dependent claims.

According to a basic concept underlying the invention, a graphical user interface consists of various elements, such as buttons, icons, boxes, values, texts etc. Such user interface elements can be active, meaning that the operator can interact with it by e.g. touching it or performing a gesture. This action triggers an event within the device. The idea of the invention is to add either a static or animated feature (e.g. coloring, blinking) to the active user interface element in order to catch the users' attention and direct it to the element, to suggest that an action is expected from the user, and/or to suggest the need for interaction with the element. Within this concept, user interface elements proactively indicate that a user interaction is required. Indication can be in form of any graphical highlighting or change, such as dynamically changing color, shape, opacity, etc.

Advantageous effects of the invention include improved usability by directing the user's attention to the user interface element that requires user action. This enhances perception, reduces cognitive load, thus speeding up user interaction and boosting user confidence. In addition, the invention can in general provide help and hints to the user on expected user actions, and an operator can be aware of which parameters are required and need to be set before proceeding to next user actions, aware that one of the buttons needs to be interacted with. Also, these advantageous effects increase the user's confidence while he uses the device, and improves the quality of involved human-machine interaction, which in turn enhances device usability.

Hereinafter, the invention will be described in more detail based on a preferred embodiment thereof and with reference to the attached drawings. In the drawings,
Fig. 1 schematically shows a flow chart of an operation flow in a blood purification device providing user interaction feedback to an active element in a graphical user interface of the device according to a preferred embodiment; and
Fig. 2 schematically exemplifies feedback presentations to a user according to the control carried out in the embodiment.

Generally referring to the preferred embodiment, the configuration thereof basically relies on supporting hardware provided in any blood purification device/machine or blood purification related device benefiting from the embodied blood purification device feedback method. Such hardware can, therefore, in particular comprise sensors, detectors, pumps, filters and various other process and measurement means of which a state or status can be queried, polled and/or sampled, or that deliver a certain value during device operation, and on suitable means arranged to process such queried status and/or values and generate an output based on the processing result.

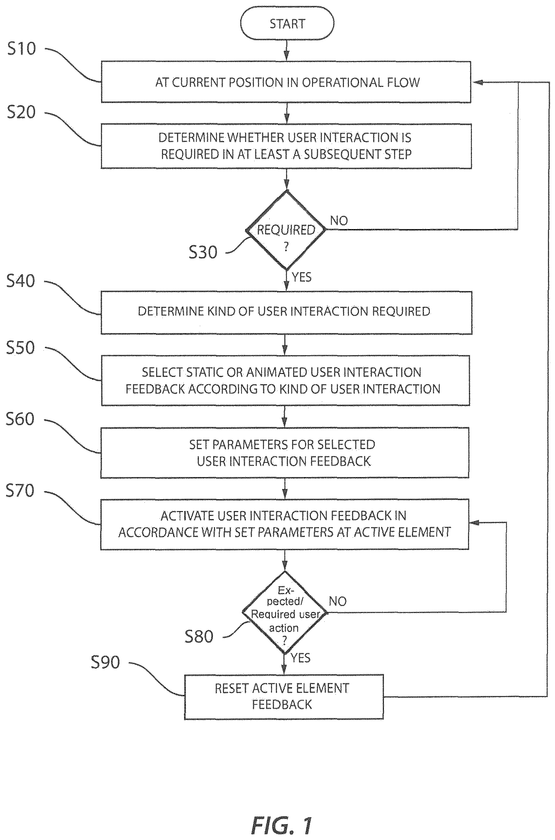

Fig. 1 schematically shows a flow chart of an operation flow in a blood purification device providing user interaction feedback to an active element in a graphical user interface of the device according to a preferred embodiment.

A processing in the underlying device monitors the operational flow thereof and branches into the flow of the present embodiment at a step S10, where a current position in the operational flow is sort of pinpointed or marked for the purpose of the present embodiment. The process then proceeds to a step S20.

In step S20, it is determined whether a user interaction is required at at least one step of the operational flow subsequent to the current step. In other words, the control proactively looks ahead, or further "down" the operational flow it is working, as to a user will be required to take any action via the graphical user interface of the device later on.

This looking ahead can for example include a predetermined number of subsequent steps and more specifically be configured to leave sufficient processing time to activate the user interaction feedback at the corresponding active element in time and without generating any waiting states, delay and the like. I.e. a user interaction feedback at the active element can be given before the device or operational flow actually requires and/or expects the user interaction to be carried out.

If no user interaction is required according to a check to this effect in a next step S30, the process returns to step S10 in order to keep track of the current position in the operational flow and to keep looking ahead the configured step range as to whether a user interaction requirement is involved in any of later operational flow positions. In other words, steps S10 to S30 are looped until a user interaction requirement is detected "down the road".

If a user interaction is required as the result of the checking in step S30, the process proceeds to a step S40, in which a kind, type, group, category etc. of the required user interaction is determined. The kind of user interaction can be used to switch and/or select between different user interaction feedback at the active element, i.e. to select a certain feedback for a certain kind of user interaction, and to select a different feedback for a different kind of user interaction. The number of distinguishable kinds of user interactions is not particularly limited but can generally correspond to the number of representable feedback indications at one or the active element(s), and it is understood that it also be only one in a more simple case. After determining the kind of user action required, the process proceeds to a step S50.

In step S50, a static or animated user interaction feedback is selected according to the kind of user interaction determined in step S40. This selecting step provides for the setting of sort of a basic type of user interaction feedback. The process then proceeds to a next step S60.

In step S60, parameters configuring the basic type of user interaction feedback selected in step S50 into a desired form.

In the present embodiment, a graphic (either static or animated) is placed on or around the button. The graphic follows the outline of the button with the sides being colored and/or animated and the middle being transparent so that the button can be seen. When animated, the animation can have various forms, and for example be represented by a fixedly colored, static frame, a gradient of colors (e.g. two colors) on a static frame, a fixedly colored, repeatedly appearing and disappearing frame, a changing color static frame, a blinking frame in which the blinking speed changes, a fixedly colored, growing and shrinking frame, and the like.

The animation as such can be used for e.g. marking parameters that need to be set (required parameters) with a fixedly colored, static frame around them, and/or marking buttons that need to be pressed as an expected next user action by a fixedly colored, repeatedly blinking frame. Indication as such can be in the form of arbitrary graphical highlighting or modification, such as dynamically changing color, shape, opacity, etc. Further modifications can change the position or size of a user interface element, or introduce other animated features (e.g. spinning).

Accordingly, in step S60, as example parameters and without limitation thereto, a color parameter defines the color in which a frame appears or what color-pairs the gradient uses as a starting and ending gradient color. A speed parameter can define the speed of an animation. When set to 0, the frame is static, and when set to other than 0, the frame is either rotating the angle of gradient or repeatedly changes an opacity value of the color from 0 to 100 and back in order to provide a blinking effect. A border weight parameter defines the number of pixels of the thickness of the frame. An enable parameter is used to control the visibility of the animation. When the enable parameter is set to 1, the animation is active and visible, and when it is set to 0, the animation is hidden.

In a subsequent step S70, the current user interaction feedback is activated at the active element in accordance with the previously set definition thereof.

After the currently applicable user interaction feedback has been set to the active element in step S70, the process proceeds to a step S80. In step S80, it is confirmed whether or not the required and/or expected user interaction has taken place, or been carried out by the user.

If it is found in step S80 that the user interaction is not completed, or has not taken place, the process returns to step S70, i.e. steps S70 and S80 are looped in order to keep the user interaction feedback at the active element alive in its current representation until the user takes action as required and/or expected. Once done, the process proceeds to a step S90.

In step S90, the user task having been carried out as required and/or expected is taken as finalized, and accordingly the user interaction feedback at the active element is reset to a predetermined non-feedback state easily recognizable as such by the user. Resetting the user interaction feedback at the active element may, for example, include turning off a lit state, halting an animation, changing a color to standard or neutral, and the like, and in connection therewith, process variables such as the determined kind of user interaction feedback, the selected type of basic representation, and any parameters varying the basic representation may also be returned to an initial state from where to suitably proceed from in a next process run.

To trigger such a next run, the process in the present embodiment is designed to jump back to step S10 from step S90, i.e. is configured as an endless loop as long as the blood purification device is operative. In this way, permanent monitoring and user interaction feedback as long as the device is operative, is achieved. It is, however, understood that the present invention is not limited to such permanent looping, and that a different process exit strategy can be used.

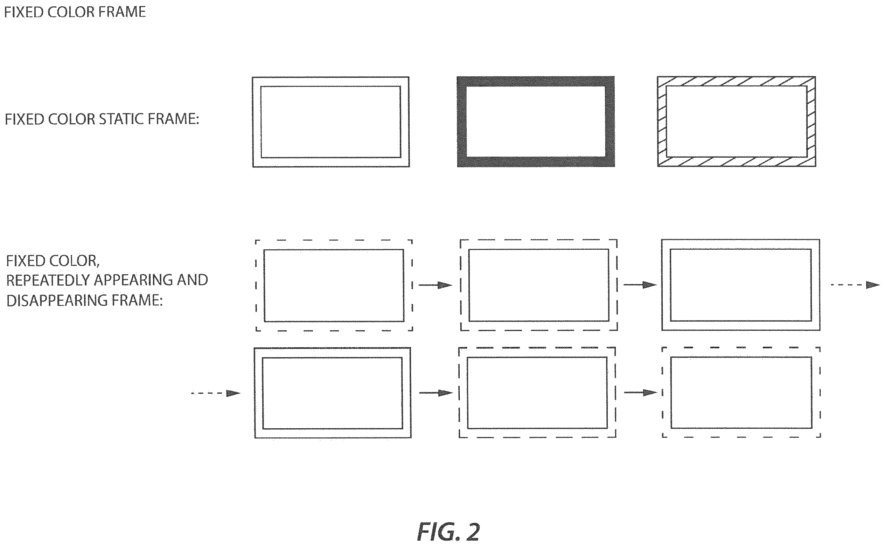

Turning to Fig. 2, feedback presentations to a user as examples of the user interaction feedback according to the control carried out in the embodiment are schematically illustrated based on a rectangle as basic form suitable for an exemplary rectangular active element or button. It is, however, understood that various representations other than a rectangle are possible.

To this effect, the upper half of Fig. 2 depicts a static frame of fixed color placed around the button as the active element. It is noted that the color is not limited to a specific one. The lower half of Fig. 2 shows a repeatedly appearing and disappearing frame of fixed color at a same button (following the arrows indicates the changes at a same button from not present/disappeared in dashed line with shorter dashed, to partially present in dashed line with longer dashes, to present/appeared in full line, and vice versa. Of course, there may be less or more intermediate stages than are shown in Fig. 2.

Insofar, along the preferred embodiment and teaching disclosed herein, the invention provides solutions for problems arising from an operator having no or little guidance on whether any user action on the graphical user interface is expected by the device, an operator having no or little guidance on which graphical user interface element needs to be interacted with, and an operator having no or little guidance on how to interact with the user interface element.

While the invention has been described with reference to a preferred embodiment and the accompanying drawings, it is understood that the present invention is not in any way limited to particular details disclosed with respect to this preferred embodiment, and that any modification readily apparent to the skilled person based on the here presented teaching is deemed to be within the scope of protection as defined by the appended claims.

## Claims

1. A method for providing feedback on a required user interaction in a blood purification device, comprising the steps of:
monitoring, at a current position in an operational flow of the blood purification device, whether a user interaction is required in at least one step of the operational flow subsequent to the current step including a predetermined number of subsequent steps to leave sufficient processing time to activate the user interaction in time without generating waiting states;
if it is determined in said monitoring step that a user interaction is required, determining the kind of required user interaction; and
proactively activating a user interaction feedback indicating the determined kind of user interaction required in said at least one subsequent step at an active element corresponding to the user interaction in a graphical user interface.

2. A method according to claim 1, comprising a step of selecting a static or an animated user interaction feedback in accordance with the kind of user interaction determined in said required user interaction determining step.

3. A method according to claim 1 or 2, wherein said proactively activating step comprises activating said user interaction feedback as a fixedly colored, static frame, a gradient of colors on a static frame, a fixedly colored and repeatedly appearing and disappearing frame, a changing color static frame, a blinking frame with variable blinking speed, and/or a fixedly colored, alternately growing and shrinking frame.

4. A method according to one of the preceding claims, comprising a step of setting at least one control parameter for said user interaction feedback, said parameters controlling an appearance of the user interaction feedback at said active element.

5. A method according to claim 3, wherein said at least one control parameter includes a color parameter defining the color in which an active element surrounding frame, as said user interaction feedback appears, and/or or color-pairs a gradient uses as a starting and ending gradient color, a speed parameter defining the speed of a user interaction feedback animation, a border weight parameter defining a thickness of the active element surrounding frame, and/or an enable parameter controlling a visibility of the user interaction feedback.

6. A method according to one of the preceding claims, comprising a step of changing the position, size and/or type of an animation of the user interaction feedback at the active element.

7. A method according to one of the preceding claims, wherein spare user interaction feedback variants not assigned to a determinable user interaction are kept available for use in an escalating step enhancing a user interaction feedback set for a determined user action beyond normal in case of occurrence of an exceptional device state.

8. A method according to one of the preceding claims, comprising the step of resetting the user interaction feedback at the active element upon detection that the determined user interaction of a completed has triggered an event in the device and/or has been carried out.

9. A blood purification device comprising components assigned to a monitorable operational flow and a graphical user interface including one or more active elements arranged to trigger one or more events in the device and to present user interaction feedback on user interaction required, based on a monitoring of said monitorable operational flow, the blood purification device being arranged to carry out the method according to one of the preceding claims.

## Patentansprüche

1. Verfahren zur Bereitstellung einer Rückmeldung über eine erforderliche Benutzerinteraktion in einer Blutreinigungsvorrichtung mit den folgenden Schritten:
Überwachen, an einer aktuellen Position in einem Betriebsablauf der Blutreinigungsvorrichtung, ob eine Benutzerinteraktion in mindestens einem Schritt des Betriebsablaufs erforderlich ist, der auf den aktuellen Schritt folgt und eine vorbestimmte Anzahl von nachfolgenden Schritten hat, um genügend Verarbeitungszeit zu lassen, um die Benutzerinteraktion rechtzeitig zu aktivieren, ohne Wartezustände zu erzeugen;
Bestimmen der Art der erforderlichen Benutzerinteraktion, wenn in dem Schritt der Überwachung festgestellt wird, dass eine Benutzerinterkation erforderlich ist; und
proaktives Aktivieren einer Benutzerinteraktionsrückmeldung, die die ermittelte Art der erforderlichen Benutzerinteraktion in dem mindestens einen nachfolgenden Schritt an einem aktiven Element anzeigt, das der Benutzerinteraktion in einer grafischen Benutzeroberfläche entspricht.

2. Verfahren gemäß Anspruch 1, mit einem Schritt des Auswählens einer statischen oder einer animierten Benutzerinteraktionsrückmeldung in Übereinstimmung mit der Art der Benutzerinteraktion, die in dem Schritt zur Bestimmung der erforderlichen Benutzerinteraktion bestimmt wurde.

3. Verfahren gemäß Anspruch 1 oder 2, wobei der Schritt des proaktiven Aktivierens das Aktivieren der Benutzerinteraktionsrückmeldung als einen fest gefärbten, statischen Rahmen, einen Farbverlauf auf einem statischen Rahmen, einen fest gefärbten und wiederholt erscheinenden und verschwindenden Rahmen, einen statischen Rahmen mit wechselnder Farbe, einen blinkenden Rahmen mit variabler Blinkgeschwindigkeit und/ oder einen fest gefärbten, abwechselnd wachsenden und schrumpfenden Rahmen hat.

4. Verfahren gemäß einem der vorhergehenden Ansprüche, mit einem Schritt des Einstellens mindestens eines Steuerparameters für die Benutzerinteraktionsrückmeldung, wobei die Parameter ein Erscheinungsbild der Benutzerinteraktionsrückmeldung an dem aktiven Element steuern.

5. Verfahren gemäß Anspruch 3, wobei der zumindest eine Steuerparameter einen Farbparameter, der die Farbe definiert, in der ein aktives Element, das den Rahmen umgibt, als die Benutzerinteraktionsrückmeldung erscheint, und/ oder einen Farbverlauf, der als Start- und End-Farbverlauf verwendet wird, einen Geschwindigkeitsparameter, der die Geschwindigkeit einer Benutzerinteraktionsrückmeldung-Animation definiert, einen Randgewichtparameter, der eine Dicke des aktiven Elements, das den Rahmen umgibt definiert, und/ oder einen Freigabeparameter hat, der eine Sichtbarkeit der Benutzerinteraktionsrückmeldung steuert.

6. Verfahren gemäß einem der vorhergehenden Ansprüche, mit einem Schritt des Änderns der Position, Größe und/ oder Art einer Animation der Benutzerinteraktionsrückmeldung am aktiven Element.

7. Verfahren gemäß einem der vorhergehenden Ansprüche, wobei Ersatzvarianten von Benutzerinteraktionsrückmeldungen, die nicht einer bestimmbaren Benutzerinteraktion zugeordnet sind, zur Verwendung in einem Eskalationsschritt zur Erweiterung eines Benutzerinteraktionsrückmeldesatzes für eine bestimmte Benutzeraktion über den Normalfall hinaus bei Auftreten eines außergewöhnlichen Gerätezustands verfügbar gehalten werden.

8. Verfahren nach einem der vorhergehenden Ansprüche, mit dem Schritt des Zurücksetzens der Benutzerinteraktionsrückmeldung am aktiven Element bei Erkennung, dass die ermittelte Benutzerinteraktion einer abgeschlossenen Benutzerinterkation ein Ereignis im Gerät ausgelöst hat und/ oder durchgeführt wurde.

9. Blutreinigungsvorrichtung mit Komponenten, die einem überwachbaren Betriebsablauf zugeordnet sind, und einer graphischen Benutzerschnittstelle, die ein oder mehrere aktive Elemente enthält, die so angeordnet sind, um ein oder mehrere Ereignisse in der Vorrichtung auszulösen und eine Rückmeldung über die erforderliche Benutzerinterkation auf der Grundlage einer Überwachung des überwachbaren Betriebsablaufs zu präsentieren, wobei die Blutreinigungsvorrichtung so angeordnet ist, um das Verfahren nach einem der vorhergehenden Ansprüche auszuführen.

## Revendications

1. Procédé de fourniture d'une rétroaction sur une interaction d'utilisateur requise dans un dispositif de purification du sang, comprenant les étapes de :
surveillance, au niveau d'une position actuelle dans un flux opérationnel du dispositif de purification du sang, si une interaction d'utilisateur est requise dans au moins une étape du flux opérationnel subséquente à l'étape actuelle incluant un nombre prédéterminé d'étapes subséquentes pour laisser un temps de traitement suffisant pour activer l'interaction d'utilisateur à temps sans générer des états d'attente ;
s'il est déterminé dans ladite étape de surveillance qu'une interaction d'utilisateur est requise, détermination du type d'interaction d'utilisateur requise ; et
activation de manière proactive d'une rétroaction d'interaction d'utilisateur indiquant le type déterminé d'interaction d'utilisateur requise dans ladite au moins une étape subséquente au niveau d'un élément actif correspondant à l'interaction d'utilisateur dans une interface utilisateur graphique.

2. Procédé selon la revendication 1, comprenant une étape de sélection d'une rétroaction d'interaction d'utilisateur statique ou animée conformément au type d'interaction d'utilisateur déterminé dans ladite étape de détermination d'interaction d'utilisateur requise.

3. Procédé selon la revendication 1 ou 2, dans lequel ladite étape d'activation de manière proactive comprend l'activation de ladite rétroaction d'interaction d'utilisateur comme un cadre statique coloré de manière fixe, un gradient de couleurs sur un cadre statique, un cadre coloré de manière fixe et apparaissant et disparaissant de manière répétitive, un cadre statique de couleur changeante, un cadre clignotant avec une vitesse de clignotement variable et/ou un cadre coloré de manière fixe et grandissant et rétrécissant de manière alternée.

4. Procédé selon l'une des revendications précédentes, comprenant une étape de réglage d'au moins un paramètre de commande pour ladite rétroaction d'interaction d'utilisateur, lesdits paramètres commandant une apparence de la rétroaction d'interaction d'utilisateur au niveau dudit élément actif.

5. Procédé selon la revendication 3, dans lequel ledit au moins un paramètre de commande inclut un paramètre de couleur définissant la couleur dans laquelle un cadre entourant l'élément actif apparaît comme ladite rétroaction d'interaction d'utilisateur, et/ou ou des paires de couleurs un gradient utilise comme une couleur de gradient de départ et de fin, un paramètre de vitesse définissant la vitesse d'une animation de rétroaction d'interaction d'utilisateur, un paramètre de poids de bord définissant une épaisseur du cadre entourant l'élément actif et/ou un paramètre d'activation commandant une visibilité de la rétroaction d'interaction d'utilisateur.

6. Procédé selon l'une des revendications précédentes, comprenant une étape de changement de la position, la taille et/ou le type d'une animation de la rétroaction d'interaction d'utilisateur au niveau de l'élément actif.

7. Procédé selon l'une des revendications précédentes, dans lequel des variantes de rétroaction d'interaction d'utilisateur de réserve non attribuées à une interaction d'utilisateur déterminable sont maintenues disponibles pour l'utilisation dans une étape de croissance améliorant une rétroaction d'interaction d'utilisateur définie pour une action d'utilisateur déterminée au-delà de la normale en cas de survenance d'un état de dispositif exceptionnel.

8. Procédé selon l'une des revendications précédentes, comprenant l'étape de réinitialisation de la rétroaction d'interaction d'utilisateur au niveau de l'élément actif lors de la détection que l'interaction d'utilisateur déterminée d'une terminée a déclenché un événement dans le dispositif et/ou a été réalisée.

9. Dispositif de purification du sang comprenant des composants attribués à un flux opérationnel pouvant être surveillé et une interface utilisateur graphique incluant un ou plusieurs éléments actifs agencés pour déclencher un ou plusieurs événements dans le dispositif et pour présenter une rétroaction d'interaction d'utilisateur sur une interaction d'utilisateur requise, sur la base d'une surveillance dudit flux opérationnel pouvant être surveillé, le dispositif de purification du sang étant agencé pour réaliser le procédé selon l'une des revendications précédentes.
